# NEW EUROPEAN PATENT SPECIFICATION

(11) **EP 1 310 173 B2**
(45) Date of publication and mention of the opposition decision: **31.12.2014**
(45) Mention of the grant of the patent: 23.05.2007
(21) Application number: 03003009.2
(22) Date of filing: 31.03.1998
(51) Int. Cl.: A23L 1/308, A23L 1/0528, A23L 1/29

(54) **Composition for providing nutrition to diabetics**
Zusammensetzung zur Ernährung von Diabetikern
Composition pour la nutrition des diabétiques

(30) Priority: 23.06.1997 EP 97201915
(43) Date of publication of application: 14.05.2003
(62) Divisional of application: 98201018.3
(73) Proprietor: Société des Produits Nestlé S.A., 1800 Vevey (CH)
(72) Inventor: Jaussan, Veronique, 94300 Vincennes (FR); Roessle, Claudia, 1110 Morges (CH); Schweizer, Thomas, 1052 Le Mont-Sur-Lausanne (CH); Bourguignon, Michel, 14740 Lasson (FR)
(74) Representative: Kingsbury, Oliver William

(56) References cited:
- EP-A- 0 504 055
- EP-A- 0 627 490
- EP-A- 0 692 252
- EP-A- 0 745 333
- EP-A- 0 756 828
- EP-A- 0 768 043
- WO-A-91/13076
- WO-A-94/12541
- FR-A- 2 673 360
- US-A- 5 292 723
- US-A- 5 531 989

## Description

### Field of the Invention

This invention relates to a nutritional composition suitable for providing nutrition to diabetic patients. The invention also relates to a method for providing nutrition to diabetic patients and to a method for the nutritional management of diabetic symptoms in patients.

### Background of the Invention

Diabetes is a general term for a group of metabolic disorders which are characterised by the inability to properly metabolise glucose. This inability is due either to a deficiency of the hormone insulin or to a resistance to the action of insulin. In either case, if untreated, the inability leads to hyperglycaemia and its complications of morbidity and mortality.

Diabetes is usually classified into three clinical classes; diabetes mellitus, gestational diabetes, and impaired glucose tolerance or glucose intolerance. Diabetes mellitus is generally broken down into two types; type I diabetes mellitus and type II diabetes mellitus. Type I diabetes mellitus occurs in individuals who produce little or no insulin and constitutes less than about 10% of the diabetic population. The onset of type I diabetes mellitus usually manifests itself during youth. Type II diabetes mellitus, or non-insulin dependent diabetes mellitus, usually develops after the age of about 30 years and constitutes more than about 90% of the diabetic population. Impaired glucose tolerance, in the clinical setting, often occurs as stress-induced hyperglycaemia.

Often, especially in severe cases, diabetes is treated by the administration of exogenous insulin or antihyperglycaemic agents. However, nutritional management of diabetes is possible and the American Diabetes Association has published guidelines for the nutritional therapy of diabetes. These guidelines suggest that diabetic patients should consume about 20 to 25 g of dietary fibre daily.

Dietary fibres may be classified as soluble and insoluble. Insoluble fibres appear to have little influence on glycemic levels. However, the incorporation of soluble fibres into foods is known reduce postprandial glycaemia in diabetics (Anderson, J.W. and Akanji, A.O.; 1993; "Treatment of Diabetes with High Fiber Diets", CRC Handbook of Dietary Fiber in Human Nutrition, CRC Press Inc, 2nd Edition, pages 443 to 470). Examples of soluble fibres which are believed to have this property are guar gum, pectin, xanthan gum, and β-glucan. This property makes soluble fibres ideal candidates for incorporation into foods for diabetics.

However, to date, soluble fibres have not been widely used in food for diabetics. A common problem is that many soluble fibres are not very palatable. Also, in the field of clinical nutrition, the incorporation of soluble fibres into enterally administered compositions is extremely difficult since soluble fibres are thickening agents and greatly increase viscosity. Consequently compositions which contain sufficient soluble fibre to significantly reduce postprandial glycaemia are usually too thick and viscous for enterally feeding; especially for patients requiring tube-feeding.

EP 0 768 043 discloses a nutritional composition for use by diabetics containing a controlled absorbed carbohadrate component, which consists of a rapidly, moderately and a slowly absorbed fraction. The composition also comprises fibre, which may be selected from any combination of fibre and which is not distiguished functionally.

EP 0 756 828 discloses a fibre composition for consumption by healthy people in Western societies, which is suitable to produce short chain fatty acids. The fibre composition comprises soluble and insoluble non-starch polysaccharides, oligosaccharides and/or resistant starch.

One nutritional composition which offers a solution to the problem is described in US patent 5,292,723. The nutritional composition described in this patent has a carbohydrate component made up of glucose polymers, modified starch, and soluble fibre. Pectin is suggested as a suitable soluble fibre. The nutritional composition has a viscosity of less than 0.05 kg/ms when measured at about 20°C. Further the nutrition composition, when consumed, results in lower glycemic response in patients as compared to glucose of the same energy content.

However there is still a need for a nutritional composition which is suitable for diabetic patients and which has good flow characteristics.

### Summary of the invention

Accordingly, this invention provides a nutritional composition for diabetic patients, the composition containing a protein source, a lipid source, and a carbohydrate source, the carbohydrate source including a fibre mixture comprising
- a viscous soluble fibre selected from the group comprising guar gum, xanthan gum, gum Arabic, pectin, β-glucan and mixtures of these, and
- inulin, a hydrolysate of inulin, or both,
wherein the nutritional composition is a liquid composition which has a viscosity, when measured at room temperature, of 0.015 to 0.03 kg/ms and in which the fibre mixture includes a source of insoluble dietary fibre and is present in an amount of 1.0 g/100 ml to 2.0 g/100 ml and the ratio of soluble fibre, including inulin, to insoluble fibre, is 1:3 to 3:1.

It is surprisingly found that the use of the fibre mixture results in an adequately reduced glycemic response while retaining reasonably low viscosity. Therefore, due to the excellent flow properties, the nutritional composition is ideally suited to be tube-fed to patients. Further, the use of inulin or its hydrolysates provides a substrate for lactic acid bacteria in the gastro-intestinal tract; leading to beneficial effects in the general health of the patient.

Preferably, the protein source provides 10% to 20% of energy, the lipid source provides 30% to 50% of energy, and the carbohydrate source provides 35% to 55% of energy.

### Detailed Description of the Preferred Embodiments

Embodiments of the invention are now described by way of example only. This invention provides a liquid nutritional composition for diabetic patients. The composition contains a protein source, a lipid source, and a carbohydrate source. The carbohydrate source includes a fibre mixture comprising a viscous soluble fibre and inulin. In this specification, the term "soluble fibre" means those dietary fibres which are characterised as soluble using the method of Prosky et al; 1988; J. Assoc. Off. Anal. Chem, **70,** 5, 1017. This is the official method of the Association of Official Analytical Chemists. The term "viscous soluble fibre" Association of Official Analytical Chemists. The term "viscous soluble fibre" means a soluble fibre which is able to increase the viscosity of the contents of the stomach and the small intestine and slow gastric emptying rates.

At viscous soluble fibres pectin and gum arabic are particularly preferred.

The inulin may be provided in the form of a natural extract which is suitable for human consumption. Extracts from chicory are particularly suitable. The extract preferably contains at least 80% by weight of inulin or a hydrolysate of inulin; more preferably at least 90% by weight of inulin or a hydrolysate of inulin. The inulin preferably has a degree of polymerisation of at least about 8; for example about 10 to about 25. Suitable inulin extracts may be obtained from Orafti SA of Tirlemont 3300, Belgium under the trade mark "Raftiline". For example, the inulin may be provided in the form of Raftiline®ST which is a fine white powder which contains about 90 to about 94% by weight of inulin, up to about 4% by weight of glucose and fructose, and about 4 to 9% by weight of sucrose. The average degree of polymerisation of the inulin is about 10 to about 12. The inulin may also be in the form of inulin hydrolysates or mixtures of inulin and inulin hydrolysates. Inulin hydrolysates are commonly known as fructooligosaccahrides or FOS.

Inulin and its hydrolysates are reported to promote the growth of bifidobacteria in the gastro-intestinal tract and, in certain circumstances prevent or decrease the growth of pathogens such as *Clostridiae.* Further, promoting the growth of bifidobacteria is reported to have various other beneficial effects. Moreover, inulin and its hydrolysates may reduce blood glucose levels.

The fibre mixture also contains a source of insoluble dietary fibre. Suitable sources of insoluble dietary fibres are hull fibres from legumes and grains; for example pea hull fibre, oat hull fibre, barley hull fibre, and soy hull fibre. Pea hull fibre is especially preferred. However any suitable source of insoluble dietary fibre may be used. These sources may also contain some soluble fibre.

The ratio of soluble fibre, including inulin, to insoluble fibre is 1:3 to 3:1; preferably about 1:1 to about 2:1. For example, ratio of soluble fibre, including inulin, to insoluble fibre is preferably about 2:1. Further, the fibre mixture is present in an amount of 1.0 g/100ml to 2.0 g/100ml; for example 1.3 g/100ml to 1.7 g/100ml; for example about 1.5 g/100 ml. In a preferred example, the fibre mixture comprises about 0.5 g/100 ml inulin, about 0.5 g/100 ml pectin or gum arabic, and about 0.5 g/100 ml outer pea fibre.

The protein source is preferably a high quality protein source; for example milk protein, whey protein, casein protein, or soy protein, or mixtures of these proteins. The protein source may be in the form of intact protein or may be hydrolyzed. Other protein sources such as rice, pea and oat protein, or mixtures thereof, may also be used. Further, if desired, the protein source may include free amino acids.

The protein source preferably provides 10% to 20% of the energy of the composition. For example, the protein source may provide about 12% to 18% of the energy of the composition; preferably about 15% of the energy of the composition.

The carbohydrate source may be any suitable carbohydrate or carbohydrate mixtures. For example, the carbohydrate source may be maltodextrin, modified starch, amylose starch, topioca starch, corn starch, or fructose, or mixtures thereof. Modified starch is preferred; especially modified topioca and corn starches. If the carbohydrate source includes maltodextrin, maltodextrin with a low DE is preferred; for example maltodextrin with a DE of 3 or less. Preferably the composition is low in or free from mono- and di-saccharides such as fructose and other exchange sugars, and lactose. For example the composition contains less than about 3 g/l of lactose.

The carbohydrate source, including the viscous soluble fibre and inulin, provides 35% to 55% of the energy of the composition; preferably 40% to 50% of the energy. For example, the carbohydrate source may provide about 45% of the energy of the composition. The amount of energy provided to the patient by the viscous soluble fibre and inulin is very small.

The lipid source is preferably rich in monounsaturated fatty acids; for example monounsaturated fatty acids may provide at least 50% of energy of the lipid source. Preferably, monounsaturated fat acids provide 25% to 35% of the energy of the composition; for example 29 to 30%. The lipid source may contain polyunsaturated fatty acids (omega-3 and omega-6 fatty acids); preferably these polyunsaturated fatty acids provide up to about 10% of the energy of the composition. For example these polyunsaturated fatty acids may provide enteral composition is preferably designed to have a polyunsaturated fatty acid omega-6 (n-6) to omega-3 (n-3) ratio of 4:1 to 10:1. Saturated fatty acids preferably provide less than 10% of the energy of the composition; for example less than about 7%.

The lipid source may provide 30% to 50% of the energy of the composition; preferably 35% to 45%. For example, the lipid source may provide about 40% of the energy of the composition.

If desired, the lipid source may include medium chain triglycerides. For example, medium chain triglycerides may make up 10% to 50% by weight of the lipid source.

Suitable sources of monounsaturated fatty acids are olive oil, sunflower oil rich in oleic acid, rapeseed oil rich in oleic acid, hazelnut oil, safflower oil, and the like. If medium chain triglycerides are included in the lipid source, fractionated coconut oils are a suitable source of medium chain triglycerides. A mixture of sunflower oil, rapeseed oil and olive oil is preferred.

The enteral composition preferably includes a complete vitamin and mineral profile. For example, sufficient vitamins and minerals may be provided to supply 75% to 250% of the recommended daily allowance of the vitamins and minerals per 1000 calories of the nutritional composition.

The nutritional composition conveniently has an osmolarity of 180 mOsm/l to 300 mOsm/l; for example 190 mOsm/l to 210 mOsm/l.

The viscosity of the nutritional composition, when measured at room temperature, is 0.015 to 0.03 kg/ms. Further, the flow rate of the nutritional composition through a standard feeding tube is preferably less than 150 minutes/l; for example less than 100 minutes/l.

The energy density of the nutritional composition is preferably 700 kcal/l to 1500 kcal/l; for example about 1000 kcal/l.

The nutritional composition is in the form of a ready-to-use formulation. In this form, the composition may be fed to a patient via a nasogastric tube, jejunum tube or by having the patient drink it. As such, the nutritional composition may be in a variety of forms; for example as a fruit juice-type beverage, a milk shake-type beverage and the like. type beverage, a milk shake-type beverage and the like.

Various flavours, sweeteners and other additives may be present. Artificial sweeteners such as acetosulfame and L-aspartyl based sweeteners may be used; for example aspartame.

The nutritional composition may be produced as is conventional; for example, by blending together the protein source, the carbohydrate source, and the lipid source. If used, the emulsifiers may be included in the blend. The vitamins and minerals may be added at this point but are usually added later to avoid thermal degradation. Any lipophilic vitamins, emulsifiers and the like may be dissolved into the lipid source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture.

The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger.

The liquid mixture may then be cooled to about 60°C to about 85°C; for example by flash cooling. The liquid mixture is then homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals. The pH and solids content of the homogenised mixture is conveniently standardised at this point.

For a product in liquid form, the homogenised mixture is preferably aseptically filled into suitable containers. Aseptic filling of the containers may be carried out by pre-heating the homogenised mixture (for example to about 75 to 85°C) and then injecting steam into the homogenised mixture to raise the temperature to about 140 to 160°C; for example at about 150°C. The homogenised mixture may then be cooled, for example by flash cooling, to a temperature of about 75 to 85°C. The homogenised mixture may then be further homogenised, cooled to about room temperature and filled into containers.

The nutritional composition may be used as a nutritional support for patients suffering from metabolic anomalies which make them susceptible to hypo- or hyperglycaemia. For example, the nutritional composition may be used as a nutritional support for patients suffering from type I diabetes mellitus, type II diabetes mellitus, or intolerance to glucose. Further, the nutritional composition may be used as a nutritional support for patients who are at risk of a recurrence of hypo- or hyperglycaemia. The nutritional composition may also be used as nutritional support for post operative patients. The nutritional composition is particularly useful for the nutritional management of diabetic symptoms of diabetic patients.

The amount of the nutritional composition required to be fed to a patient will vary depending upon factors such as the patient's condition, the patient's body weight, the age of the patient, and whether the nutritional composition is the sole source of nutrition. However the required amount may be readily set by a medical practitioner. In general, sufficient of the nutritional composition is administered to provide the patient with about 1 g protein to about 4.0 g protein per kg of body weight per day. For example, an adult patient may be administered about 1.5 g protein to about 2.0 g protein per kg of body weight per day. Further, sufficient of the nutritional composition is administered to provide the patient with up to about 40 g of dietary fibre (insoluble and soluble) per day; for example about 25 g to about 35 g of dietary fibre per day. If the nutritional formula is used as a supplement to other foods, the amount of the nutritional composition that is administered daily may be decreased accordingly.

The nutritional composition may be taken in multiple doses, for example 2 to 5 times, to make up the required daily amount or may taken in a single dose. The nutritional composition may also be fed continuously over a desired period.

### Example 1

A ready-to-use nutritional composition is prepared. The nutritional composition includes the following components:

| **Component** | **Conc. (/100ml)** | **Energy (%)** |
|---|---|---|
| Protein | 3.8 g | 15 |
| Casein/Soy Protein (1:1) | | |
| Carbohydrate | 11.2 g | 45 |
| Maltodextrin (low DE) | 1.0 g | |
| Modified Starch | 10.2 g | |
| Soluble fibre including Inulin | 1.0 g | |
| Insoluble fibre | 0.5 g | |
| Lipids | 4.4 g | 40 |
| Rapeseed oil, Sunflower oil, Olive oil, Glyceryl stearate, Soya lecithin | | |
| Vitamins | | |
| Vitamin A | 150 IU | |
| Vitamin C | 10 mg | |
| Vitamin D | 10 IU | |
| Vitamin E | 1.0 mg | |
| Vitamin K | 3.0 µg | |
| Thiamin | 0.1 mg | |
| Riboflavine | 0.12 mg | |
| Pantothenic acid | 0.50 mg | |
| Vitamin B6 | 0.14 mg | |
| Vitamin B 12 | 0.30 µg | |
| Niacin | 1.2 mg | |
| Folic acid | 18 µg | |
| Biotin | 10 µg | |
| Minerals | | |
| Zinc, Iron, Copper, Magnesium, Manganese, Selenium, Iodine, Potassium, Calcium, Phosphorous, Chloride | | |

The composition has an osmolarity of 210 mOsm/l and an osmolality of 240 mOsm/kg. The viscosity is 0.023 kg/ms and the free flow rate is less than 70 minutes per 500 ml through standard enteral tubing.

### Example 2

Eight healthy volunteers, of both sexes, are used in the study. The volunteers are between 20 and 45 years of age and have fasting blood glucose levels of about 70 to about 110 mg/dl. Any volunteer exhibiting the symptoms of diabetes mellitus type I or II or fructose intolerance are excluded.

The study is carried out in two stages, each stage comprising two study days separated by a wash out period of at least 7 days. The stages are also separated by a wash out period of at least 7 days. Prior to each study day, each volunteer consumes an evening meal of pizza, salad and an apple. No alcohol is taken. Then, from 10 pm, each volunteer undergoes an overnight fast. In the morning of the study day, an indwelling catheter is placed on each volunteer. A blood sample is then taken. A meal is then consumed within 10 minutes of the taking of the sample. Further blood samples are taken at 15, 30, 45, 60, 90, 120 and 180 minutes after meal intake. The blood glucose for each sample is analysed by the glucose oxidase method using a COBAS analyser (Hoffmann-La Roche). The insulin level for each sample is measured by radioimmune assay (Pharmacia).

Blood glucose levels are analysed using a two-way ANOVA. The integrated area under the curve (AUC) is calculated using the method of Wolever et al; 1986; Am. J. Clin. Nutr., 43, 167-172. The difference between the AUC of the various curves is evaluated using a Wilcoxon matched pairs test or the Hill Armitage test for cross-over design. The glycemic response is considered as different if the AUC curves are statistically different with p<0.05.

Three products are used as the meals; the product of example 1, the Sondalis® Fiber product of Nestlé Clinical Nutrition, and the Fresubin Diabetes product of Fresenius GmbH. Four hundred 400 ml of the product of example 1, 400 ml of the Sondalis® Fiber product, and 444 ml of the Fresubin Diabetes product are consumed in each case to provide a standard energy intake of 400 kcal. All meals contain comparable amounts of minerals and micronutrients.

On the first study day of stage 1, 4 volunteers are fed the product of example 1 and 4 volunteers are fed the Sondalis® Fiber product. The selection of volunteers for any product is random. On the second day of stage 1, the products are reversed. On the first day of stage 2, 4 volunteers are fed the product of example 1 and 4 volunteers are fed the Fresubin Diabetes product. The selection of volunteers for any product is random. On the second day of stage 2, the products are reversed.

The results are as follows:

| Time (Minutes) | Plasma Glucose Mean (mmol/l) | | |
|---|---|---|---|
| | Product Example 1 | Sondalis® Fiber Product | Fresubin Diabetes product |
| -10 | 4.88 | 4.85 | 5.01 |
| 15 | 5.24 | 5.56 | 5.36 |
| 30 | 5.21 | 6.29 | 6.31 |
| 45 | 4.70 | 5.28 | 5.65 |
| 60 | 4.43 | 4.80 | 4.60 |
| 90 | 4.35 | 4.84 | 4.28 |
| 120 | 4.64 | 4.22 | 4.32 |
| 180 | 4.47 | 4.06 | 4.47 |

The area under the curve results are as follows:

| | Product Example 1 | Sondalis® Fiber Product | Fresubin Diabetes product |
|---|---|---|---|
| Area at 120 minutes (mmol/l) | 37 | 84 | 49 |

The glycemic responses of the product of example 1 and the Fresubin Diabetes product are significantly lower than that of the Sondalis® Fiber; about 30 mmol/l and 50 mmol/l respectively after 120 minutes compared to about 80 mmol/l after 120 minutes. Further, for the product of example 1, blood glucose response is much flatter than that of the Sondalis® Fiber product. The peak change of the product of example 1 is also less than that of the other products. While the glycemic response of the product of example 1 is lower than that of the Fresubin Diabetes product, the differences are not significant. Therefore the product of example 1 is suitable for use with diabetic patients.

None of the patients indicated any symptoms of digestive intolerance.

### Example 3

The viscosity and flow rate of the composition of example 1 and the product sold by Fresenius GmbH under the name Fresubin DFN Plus are determined:

The viscosity of each composition is determined at 25°C using a Contraves Rheomat according to manufacturer's instructions. The composition of example 1 is filled into a sealed, flexible bag and connected to a stand at a standard height. The Fresubin DFN Plus product, which is in a glass container (its original packing) is connected to a separate stand at the same height. An enteral feeding tube is connected to each container and the time required to deliver 500 ml through the enteral feeding tube is determined. An open reservoir is then connected to each stand and the composition of example 1 poured into one reservoir and the Fresubin DFN Plus product poured into the other. An enteral feeding tube is connected to each reservoir and the time required to deliver 500 ml through the enteral feeding tube is determined.

| **Property** | **Composition of example 1** | **Fresubin DFN Plus product** |
|---|---|---|
| Viscosity (kg/ms) | 0.023 | 0.035 |
| Free Flow Rate (min/500ml) | | |
| - Original Container | 66 | 158 |
| - Open reservoir | 60 | 129 |

The results indicate that the composition of example 1 has a superior flow rate.

### Example 4

A ready-to-use nutritional composition is prepared. The nutritional composition includes the following components:

| **Component** | **Conc. (/100ml)** | **Energy (%)** |
|---|---|---|
| Protein | 3.8 g | 15 |
| Casein/Soy Protein (1:1) | | |
| Carbohydrate | 11.2 g | 45 |
| Maltodextrin (low DE) | 1.0 g | |
| Modified topioca and corn starch | 10.2 g | |
| Soluble fibre including Inulin | 1.0 g | |
| Insoluble fibre | 0.5 g | |
| Lipids | 4.4 g | 40 |
| Rapeseed oil, Sunflower oil, Olive oil, Mono- and di-glycerides (E471) | | |
| Vitamins and minerals as in example 1 | | |

The composition has an osmolarity of 190 mOsm/l. The viscosity is about 0.023 kg/ms and the free flow rate is less than about 70 minutes per 500 ml through standard enteral tubing. Monounsaturated fatty acids provide about 29% of energy, polyunsaturated fatty acids provide about 6% of energy, and saturated fatty acids provide about 5% of energy.

## Claims

1. A nutritional composition for diabetic patients, the composition containing a protein source, a lipid source, and a carbohydrate source, the carbohydrate source including a fibre mixture comprising
- a viscous soluble fibre selected from guar gum, xanthan gum, gum Arabic, pectin, β-glucan and mixtures of these, and
- inulin, a hydrolysate of inulin, or both,
wherein the nutritional composition is a liquid composition which has a viscosity, when measured at room temperature, of 0.015 to 0.03 kg/ms and in which the fibre mixture includes a source of insoluble dietary fibre and is present in an amount of 1.0 g/100ml to 2.0 g/100ml and the ratio of soluble fibre, including inulin, to insoluble fibre, is 1:3 to 3:1.

2. The composition according to Claim 1, wherein the protein source provides 10% to 20% of the energy, the lipid source provides 30% to 50% of the energy, and the carbohydrate source provides 35% to 55% of the energy of the nutritional composition.

3. The nutritional composition according to claim 2 in which the source of insoluble dietary fibre is pea hull fibre.

4. The nutritional composition according to any of claims 1 to 3 in which the fibre mixture comprises about 0.5 g/100 ml inulin, about 0.5 g/100 ml pectin or gum arabic, and about 0.5 g/100 ml pea hull fibre.

5. The nutritional composition according to any of claims 1 to 4 in which the lipid source includes monounsaturated fatty acids which provide 25% to 35% of the energy of the composition.

6. The nutritional composition according to any of claims 1 to 5 in which the lipid source further includes saturated fatty acids providing less than 10% of the energy of the composition.

## Patentansprüche

1. Nährstoffzusammensetzung für Diabetespatienten, wobei die Zusammensetzung eine Proteinquelle, eine Lipidquelle und eine Kohlenhydratquelle enthält, wobei die Kohlenhydratquelle eine Ballaststoffmischung einschließt, die Folgendes umfasst:
einen viskosen, löslichen Ballaststoff, der aus Guar-Gummi, Xanthan-Gummi, Gummiarabikum, Pektin, β-Glucan und Mischungen aus diesen ausgewählt ist, und
Inulin, ein Hydrolysat von Inulin oder beides,
worin die Nährstoffzusammensetzung eine flüssige Zusammensetzung ist, die eine Viskosität, bei Messung bei Raumtemperatur, von 0,015 bis 0,03 kg/ms aufweist und in der die Ballaststoffmischung eine Quelle an unlöslichem Ballaststoff einschließt und in einer Menge von 1,0 g/100 ml bis 2,0 g/100 ml vorliegt und das Verhältnis von löslichem Ballaststoff, einschließlich Inulin, zu unlöslichem Ballaststoff 1:3 bis 3:1 beträgt.

2. Zusammensetzung nach Anspruch 1, worin die Proteinquelle 10 % bis 20 % der Energie bereitstellt, die Lipidquelle 30 % bis 50 % der Energie bereitstellt und die Kohlenhydratquelle 35 % bis 55 % der Energie der Nährstoffzusammensetzung bereitstellt.

3. Nährstoffzusammensetzung nach Anspruch 2, in der die Quelle des unlöslichen Ballaststoffs Erbsenhülsenfaser ist.

4. Nährstoffzusammensetzung nach einem der Ansprüche 1 bis 3, in der die Ballaststoffmischung etwa 0,5 g/100 ml Inulin, etwa 0,5 g/100 ml Pektin oder Gummiarabikum und etwa 0,5 g/100 ml Erbsenhülsenfaser umfasst.

5. Nährstoffzusammensetzung nach einem der Ansprüche 1 bis 4, in der die Lipidquelle einfach ungesättigte Fettsäuren einschließt, die 25 % bis 35 % der Energie der Zusammensetzung bereitstellen.

6. Nährstoffzusammensetzung nach einem der Ansprüche 1 bis 5, in der die Lipidquelle weiter gesättigte Fettsäuren einschließt, die weniger als 10 % der Energie der Zusammensetzung bereitstellen.

## Revendications

1. Composition nutritionnelle pour patients diabétiques, la composition contenant une source de protéines, une source de lipides, et une source de glucides, la source de glucides incluant un mélange de fibres comprenant
- une fibre soluble visqueuse sélectionnée parmi de la gomme de guar, de la gomme de xanthane, de la gomme arabique, de la pectine, du β-glucane et des mélanges de ceux-ci, et
- de l'inuline, un hydrolysat d'inuline, ou les deux,
dans laquelle la composition nutritionnelle est une composition liquide qui a une viscosité, quand mesurée à la température ambiante, de 0,015 à 0,03 kg / ms et dans laquelle le mélange de fibres inclut une source de fibre diététique insoluble et est présente dans une quantité de 1,0 g / 100 ml à 2,0 g / 100 ml et le rapport de fibre soluble, y compris l'inuline, sur la fibre insoluble, est 1 : 3 à 3 : 1.

2. Composition selon la revendication 1, dans laquelle la source de protéines fournit 10 % à 20 % de l'énergie, la source de lipides fournit 30 % à 50 % de l'énergie, et la source de glucides fournit 35 % à 55 % de l'énergie de la composition nutritionnelle.

3. Composition nutritionnelle selon la revendication 2, dans laquelle la source de fibre diététique insoluble est une fibre de coques de pois.

4. Composition nutritionnelle selon l'une quelconque des revendications 1 à 3, dans laquelle le mélange de fibres comprend environ 0,5 g / 100 ml d'inuline, environ 0,5 g / 100 ml de pectine ou de gomme arabique, et environ 0,5 g / 100 ml de fibre de coques de pois.

5. Composition nutritionnelle selon l'une quelconque des revendications 1 à 4, dans laquelle la source de lipides inclut des acides gras mono-insaturés qui fournissent 25 % à 35 % de l'énergie de la composition.

6. Composition nutritionnelle selon l'une quelconque des revendications 1 à 5, dans laquelle la source de lipides inclut en outre des acides gras saturés fournissant moins de 10 % de l'énergie de la composition.
